# EUROPEAN PATENT APPLICATION

(11) **EP 2 281 819 A1**
(43) Date of publication of application: **09.02.2011**
(21) Application number: 10166506.5
(22) Date of filing: 20.01.2005
(51) Int. Cl.: C07D 263/58, C07D 235/30, C07D 498/04, C07D 487/04

(54) **Benzimidazolyl or benzoxazolyl derivatives**

(30) Priority: 21.01.2004 GB 0401334
(62) Divisional of application: 05701081.1
(71) Applicant: Novartis AG, 4056 Basel (CH)
(72) Inventor: Press, Neil John, Horsham, West Sussex, Sussex RH12 5AB (GB); Porter, David, Horsham, West Sussex, Sussex RH12 5AB (GB); Leblanc, Catherine, Horsham, West Sussex, Sussex RH12 5AB (GB); McCarthy, Clive, 4002, Basel (CH); Soldermann, Nils, 4002, Basel (CH); Brown, Lyndon Nigel, Horsham, West Sussex, Sussex RH12 5AB (GB)
(74) Representative: Vögeli-Lange, Regina

(57) **Abstract**

Compounds of formula I 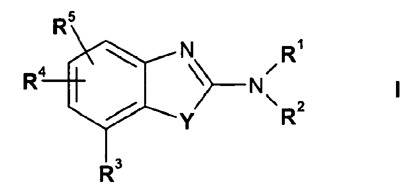 in free or salt form, where Y, R¹, R², R³, R⁴ and R⁵ have the meanings as indicated in the specification, are useful for treating conditions that are mediated by the CXCR2 receptor. Pharmaceutical compositions that contain the compounds and a process for preparing the compounds are also described.

## Description

This invention relates to organic compounds, their preparation and use as pharmaceuticals.

In one aspect the invention provides compounds of formula I 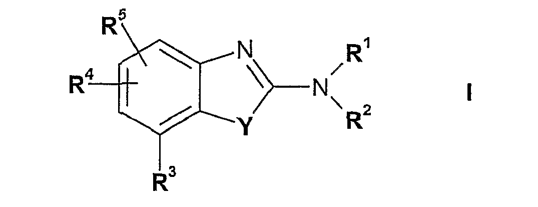 in free or salt form, where
Y is nitrogen or oxygen;
R¹ is hydrogen or C₁-C₈-alkyl;
R² is a C₃-C₁₅-carbocyclic group optionally substituted by halo, cyano, hydroxy, carboxy, nitro, aminocarbonyl, C₁-C₈-alkyl or by C₁-C₈-alkoxy optionally substituted by a 5- or 6-membered heterocyclic group containing at least one ring heteroatom selected from nitrogen, oxygen and sulphur,
or R² is C₁-C₈-alkyl or C₁-C₈-alkoxy in either case being optionally substituted by hydroxy, carboxy, -SO₂NH₂, C₁-C₈-alkylsulfonylamino or a 5- or 6-membered heterocyclic group containing at least one ring heteroatom selected from nitrogen, oxygen and sulphur,
or R² is C₁-C₈-alkylcarbonyl, C₃-C₈-cycloalkyl or a 5- or 6-membered heterocyclic group containing at least one ring heteroatom selected from nitrogen, oxygen and sulphur,
or R¹ and R² together form C₃-C₈-cycloalkyl or a 5- or 6-membered heterocyclic group containing at least one ring heteroatom selected from nitrogen, oxygen and sulphur;
R³ is hydrogen, halo, cyano, hydroxy, nitro, carboxy, aminocarbonyl, C₁-C₈-alkyl, C₁-C₈-alkenyl, C₁-C₈-alkynyl, C₁-C₈-haloalkyl, C₁-C₈-alkylthio, C₃-C₈-cycloalkyl, C₃-C₈-cycloalkyl-C₁-C₈-alkyl, carboxy-C₁-C₈-alkoxy, -CO-NR⁶R⁷, -NH-SO₂R⁸, -NH-COH or -SO₂NH₂,
and R⁴ and R⁵ are independently hydrogen, halo, cyano, hydroxy, nitro, carboxy, aminocarbonyl, C₁-C₈-alkyl, C₁-C₈-alkenyl, C₁-C₈-alkynyl, C₁-C₈-haloalkyl, C₁-C₈-alkylthio, C₁-C₈-alkoxy, C₁-C₈-haloalkoxy, C₃-C₈-cycloalkyl, C₃-C₈-cycloalkyl-C₁-C₈-alkyl, C₃-C₈-cycloalkyl-oxy, -CO-NR⁶R⁷, -NH-SO₂R⁸, -NH-COH or -SO₂NH₂,
or two of R³, R⁴ and R⁵ that are attached to adjacent ring carbon atoms together form a 5- or 6-membered heterocyclic group containing at least one ring heteroatom selected from nitrogen, oxygen and sulphur; and
R⁶, R⁷ and R⁸ are independently hydrogen or C₁-C₈-alkyl.

Terms used in the specification have the following meanings:
"Optionally substituted" means the group referred to can be substituted at one or more positions by any one or any combination of the radicals described.
"C₁-C₈-alkyl" as used herein denotes straight chain or branched alkyl having 1 to 8 carbon atoms. Preferably, C₁-C₈-alkyl is C₁-C₄-alkyl.
"C₂-C₈-alkenyl" as used herein denotes straight chain or branched hydrocarbon chains that contain two to eight carbon atoms and one or more carbon-carbon double bonds. Preferably "C₂-C₈-alkenyl" is "C₂-C₄-alkenyl".
"C₂-C₈-alkynyl" as used herein denotes straight chain or branched hydrocarbon chains that contain two to eight carbon atoms and one or more carbon-carbon triple bonds. Preferably "C₂-C₈-alkynyl" is "C₂-C₄-alkynyl".
"C₃-C₈-cycloalkyl" as used herein may be, for example, cyclopropyl, cyclobutyl, cyclopentyl, methylcyclopentyl, cyclohexyl, methylcyclohexyl, dimethylcyclohexyl, cycloheptyl, bicycloheptyl, cyclooctyl and bicyclooctyl. Preferably "C₃-C₈-cycloalkyl" is "C₃-C₆-cycloalkyl". ,
"C₃-C₈-cycloalkyl-C₁-C₈-alkyl" as used herein denotes C₁-C₈-alkyl as hereinbefore defined substituted by C₃-C₈-cycloalkyl as hereinbefore defined. Preferably "C₃-C₈-cydoalkyl-C₁-C₈-alkyl" is "C₃-C₆-cycloalkyl-C₁-C₄-alkyl".
"C₃-C₁₅-carbocyclic group" as used herein denotes a carbocyclic group having 3 to 15 ring carbon atoms, for example a monocyclic group, either aromatic or non-aromatic, such as a cyclopropyl, cyclobutyl, cyclopentyl, cyclohexyl, cycloheptyl, cyclooctyl or phenyl, which can be substituted by one or more, usually one or two, C₁-C₄-alkyl groups, or a bicyclic group such as bicyclooctyl, bicyclononyl including indanyl and indenyl, and bicyclodecyl including naphthyl, again any of which can be substituted by one or more, usually one or two, C₁-C₄-alkyl groups. Preferably the C₃-C₁₅-carbocyclic group is a C₅-C₁₀-carbocyclic group, for example phenyl or naphthyl.
"C₃-C₈-cycloalkyl-oxy" as used herein denotes C₃-C₈-cycloalkyl as hereinbefore defined linked to an oxo group. Preferably "C₃-C₈-cydoalkyl-oxy" is "C₃-C₆-cycloalkyl-oxy".
"C₁-C₈-haloalkyl" as used herein denotes C₁-C₈-alkyl as hereinbefore defined substituted by one or more halogen atoms, preferably one, two or three halogen atoms. Preferably "C₁-C₈-haloalkyl" is "C₁-C₄-haloalkyl".
"C₁-C₈-alkylcarbonyl" as used herein denotes C₁-C₈-alkyl as hereinbefore defined linked to a carbonyl group. Preferably "C₁-C₈-alkylcarbonyl" is "C₁-C₄-alkylcarbonyl".
"C₁-C₈-alkylthio" as used herein denotes C₁-C₈-alkyl as hereinbefore defined linked to -S-. Preferably "C₁-C₈-alkylthio" is "C₁-C₄-alkylthio".
"C₁-C₈-alkylsulfonylamino" as used herein denotes C₁-C₈-alkyl as hereinbefore defined linked to -SO2-NH-. Preferably "C₁-C₈-alkylsulfonylamino" is "C₁-C₄-alkylsulfonylamino".
"C₁-C₈-alkoxy" as used herein denotes straight chain or branched alkoxy having 1 to 8 carbon atoms. Preferably, C₁-C₈-alkoxy is C₁-C₄-alkoxy.
"C₁-C₈-haloalkoxy" as used herein denotes C₁-C₈-alkoxy as hereinbefore defined substituted by one or more halogen atoms, preferably one, two or three halogen atoms. Preferably "C₁-C₈-haloalkoxy" is "C₁-C₄-haloalkoxy".
"Carboxy-C₁-C₈-alkoxy" as used herein denotes C₁-C₈-alkoxy as hereinbefore defined substituted by one or more carboxy groups, preferably one or two carboxy groups. Preferably "carboxy-C₁-C₈-alkoxy" is "carboxy-C₁-C₄-alkoxy".
"Halo" or "halogen" as used herein denotes a element belonging to group 17 (formerly group VII) of the Periodic Table of Elements, which may be, for example, fluorine, chlorine, bromine or iodine. Preferably halo or halogen is chlorine or bromine.
"Aminocarbonyl" as used herein denotes amino attached through the nitrogen atom to a carbonyl group.
"5- or 6- membered heterocyclic group containing at least one ring heteroatom selected from nitrogen, oxygen and sulphur" as used herein may be, for example, pyrrole, pyrrolidine, pyrazole, imidazole, triazole, tetrazole, thiadiazole, isothiazole, oxadiazole, pyridine, oxazole, isoxazole, pyrazine, pyridazine, pyrimidine, piperazine, morpholino, triazine, oxazine or thiazole. The heterocyclic group, which is preferably aromatic, can be unsubstituted or substituted. Preferred substituents include halo, cyano, hydroxy, carboxy, nitro, amido, C₁-C₈-alkyl, and C₁-C₈-alkoxy optionally substituted by aminocarbonyl.

Throughout this specification and in the claims that follow, unless the context requires otherwise, the word "comprise", or variations such as "comprises" or "comprising", will be understood to imply the inclusion of a stated integer or step or group of integers or steps but not the exclusion of any other integer or step or group of integers or steps.

In a first aspect of the invention preferred compounds include those of formula I in free or salt form, where
Y is nitrogen or oxygen;
R¹ is hydrogen;
R² is a C₃-C₁₆-carbocyclic group optionally substituted by halo, C₁-C₈-alkyl or by C₁-C₈-alkoxy optionally substituted by a 5- or 6-membered heterocyclic group containing at least one ring heteroatom selected from nitrogen, oxygen and sulphur; and
R³, R⁴ and R⁵ are independently hydrogen, halo, cyano, hydroxy, carboxy, -SO₂NE₂, C₁-C₈-alkoxy or -CO-NH₂,
or two of R³, R⁴ and R⁵ that are attached to adjacent ring carbon atoms together form a 5- or 6-membered heterocyclic group containing at least one ring heteroatom selected from nitrogen, oxygen and sulphur.

Especially preferred compounds include those of formula I in free or salt form, where
Y is nitrogen or oxygen;
R¹ is hydrogen;
R² is a C₃-C₁₀-carbocyclic group, preferably phenyl or naphthyl, optionally substituted by halo, C₁-C₄-alkyl or by C₁-C₄-alkoxy optionally substituted by a 5- or 6-membered heterocyclic group containing at least one ring heteroatom selected from nitrogen, oxygen and sulphur; and R³, R⁴ and R⁵ are independently hydrogen, halo, cyano, hydroxy, carboxy, -SO₂NE₂, C₁-C₄-alkoxy or -CO-NH₂,
or two of R³, R⁴ and R⁵ that are attached to adjacent ring carbon atoms together form a 5- or 6-membered heterocyclic group containing at least one ring heteroatom selected from nitrogen, oxygen and sulphur.

In a second aspect of the invention preferred compounds include those of formula I in free or salt form, where
Y is nitrogen or oxygen;
R¹ is hydrogen;
R² is a C₃-C₁₅-carbocyclic group optionally substituted by halo, C₁-C₈-alkyl or by C₁-C₈-alkoxy optionally substituted by a 5- or 6-membered heterocyclic group containing at least one ring heteroatom selected from nitrogen, oxygen and sulphur; and
R³, R⁴ and R⁵ are independently hydrogen, halo, cyano, hydroxy, carboxy or -SO₂NH₂,
or two of R³, R⁴ and R⁵ that are attached to adjacent ring carbon atoms together form a 5- or 6-membered heterocyclic group containing at least one ring heteroatom selected from nitrogen, oxygen and sulphur.

Especially preferred compounds include those of formula I in free or salt form, where
Y is nitrogen or oxygen;
R¹ is hydrogen;
R² is a C₃-C₁₀-carbocyclic group, preferably phenyl or naphthyl, optionally substituted by halo, C₁-C₄-alkyl or by C₁-C₄-alkoxy optionally substituted by a 5- or 6-membered heterocyclic group containing at least one ring heteroatom selected from nitrogen, oxygen and sulphur; and R³, R⁴ and R⁵ are independently hydrogen, halo, cyano, hydroxy, carboxy or -SO₂NH₂, or two of R³, R⁴ and R⁵ that are attached to adjacent ring carbon atoms together form a 5- or 6-membered heterocyclic group containing at least one ring heteroatom selected from nitrogen, oxygen and sulphur.

The compounds represented by formula I can form acid addition salts, particularly pharmaceutically acceptable acid addition salts. Pharmaceutically acceptable acid addition salts of the compound of formula I include those of inorganic acids, for example, hydrohalic acids such as hydrofluoric acid, hydrochloric acid, hydrobromic acid or hydroiodic acid, nitric acid, sulfuric acid, phosphoric acid; and organic acids, for example aliphatic monocarboxylic acids such as formic acid, acetic acid, trifluoroacetic acid, propionic acid and butyric acid, aliphatic hydroxy acids such as lactic acid, citric acid, tartaric acid or malic acid, dicarboxylic acids such as maleic acid or succinic acid, aromatic carboxylic acids such as benzoic acid, p-chlorobenzoic acid, diphenylacetic acid or triphenylacetic acid, aromatic hydroxy acids such as o-hydroxybenzoic acid, p-hydroxybenzoic acid, 1-hydroxynaphthalene-2-carboxylic acid or 3-hydroxynaphthalene-2-carboxylic acid, and sulfonic acids such as methanesulfonic acid or benzenesulfonic acid. These salts may be prepared from compounds of formula I by known salt-forming procedures.

Compounds of formula I which contain acidic, e.g. carboxyl, groups, are also capable of forming salts with bases, in particular pharmaceutically acceptable bases such as those well known in the art; suitable such salts include metal salts, particularly alkali metal or alkaline earth metal salts such as sodium, potassium, magnesium or calcium salts, or salts with ammonia or pharmaceutically acceptable organic amines or heterocyclic bases such as ethanolamines, benzylamines or pyridine. These salts may be prepared from compounds of formula I by known salt-forming procedures.

Specific especially preferred compounds of the invention are those described hereinafter in the Examples.

The invention also provides a process for the preparation of compounds of formula I which comprises
(i)
   (A) for the preparation of compounds of formula I where Y is nitrogen, cyclising a compound of formula IIa or IIb in either case optionally in protected form 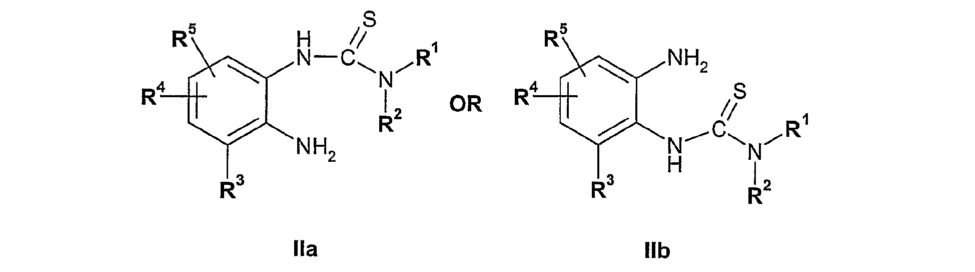 where R¹, R², R³, R⁴ and R⁵ are as hereinbefore defined, and deprotecting where necessary; or
   (B) for the preparation of compounds of formula I where Y is oxygen, cyclising a compound of formula IIc optionally in protected form 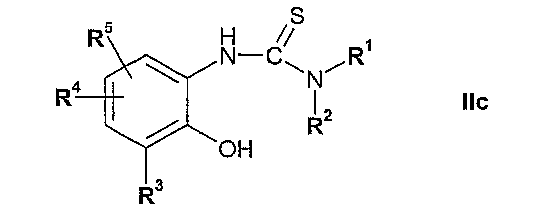 where R¹, R², R³, R⁴ and R⁵ are as hereinbefore defined, and deprotecting where necessary; and
(ii) recovering the product in free or salt form.

Process variant (A) may be effected using known procedures for cyclising o-aminophenyl-thioureas to form benzoimidazoles or analogously as hereinafter described in the Examples. The reaction is conveniently carried out in an organic solvent, for example ethanol in the presence of a coupling agent, for example 1-(-3-dimethylamino-propyl)-3-ethylcarbodiimide hydrochloride (EDCI). The reaction is carried out at an elevated temperature, for example between 70° C and 120° C, but conveniently at about 80° C or reflux temperature.

Process variant (B) may be effected using known procedures for cyclising o-hydroxyphenyl-thioureas to form benzoxazoles or analogously as hereinafter described in the Examples. The reaction is conveniently carried out in an organic solvent, for example ethanol in the presence of a coupling agent, for example EDCI. The reaction is carried out at an elevated temperature, for example between 70°C and 120°C, but conveniently at about 80° C or reflux temperature.

Compounds of formula IIa or IIb may be prepared by reacting a compound of formula IIIa or IIIb respectively 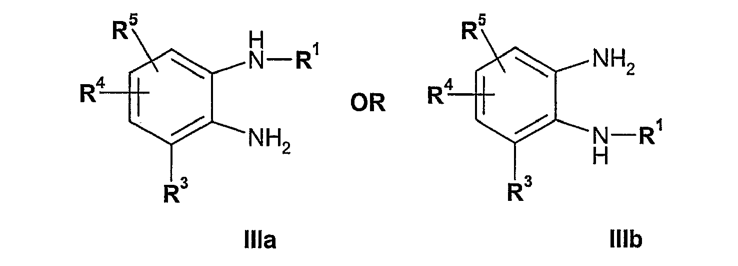 where R¹, R³, R⁴ and R⁵ are as hereinbefore defined, with a compound of formula IV

S=C=N-**R**² **IV**

using known methods for condensing aromatic diamines with thioisocyanates, or analogously as hereinafter described in the Examples. The reaction is conveniently carried out in an organic solvent, for example ethanol. Suitable reaction temperatures are from 10° C to 40° C, for example room temperature.

Compounds of formula IIc may be prepared by reacting a compound of formula IIIc 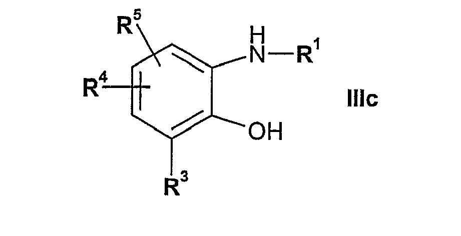 where R¹, R³, R⁴ and R⁵ are as hereinbefore defined, with a compound of formula IV using known methods for condensing aromatic amino-phenols with thioisocyanates, or analogously as hereinafter described in the Examples. The reaction is conveniently carried out in an organic solvent, for example ethanol. Suitable reaction temperatures are from 10° C to 40° C, for example room temperature.

Compounds of formula IIIa or IIIb are known or may be prepared by reacting the corresponding nitro-phenylamine with a suitable reducing agent.

Compounds of formula IIIc are known or may be prepared by known procedures, for example by reacting the corresponding nitro-phenylamine with a suitable reducing agent.

Compounds of formula IV are known or may be prepared by known procedures.

Where reference is made herein to protected functional groups or to protecting groups, the protecting groups may be chosen in accordance with the nature of the functional group, for example as described in Protective Groups in Organic Synthesis, T.W. Greene and P.G.M. Wuts, John Wiley & Sons Inc, Third Edition, 1999, which reference also describes procedures suitable for replacement of the protecting groups by hydrogen.

Compounds of formula I in free form may be converted into salt form, and vice versa, in a conventional manner. The compounds in free or salt form can be obtained in the form of hydrates or solvates containing a solvent used for crystallization. Compounds of formula I can be recovered from reaction mixtures and purified in a conventional manner. Isomers, such as enantiomers, may be obtained in a conventional manner, e.g. by fractional crystallization or asymmetric synthesis from correspondingly asymmetrically substituted, e.g. optically active, starting materials.

Compounds of formula I in free or pharmaceutically acceptable salt form, hereinafter referred to alternatively as agents of the invention, are useful as pharmaceuticals. Accordingly the invention also provides a compound of formula I in free or pharmaceutically acceptable salt form for use as a pharmaceutical. The agents of the invention act as CXCR2 receptor antagonists, thereby inhibiting the infiltration and activation of inflammatory cells, in particular neutrophils, monocytes and CD8+ T cells and mediators involved in chronic obstructive pulmonary disease (COPD). The agents of the invention therefore provide symptomatic relief and reduce disease progression.

The airways of subject with COPD exhibit an inflammatory response which is predominantly neutrophilic. When the airways are exposed to cigarette smoke macrophages, CD8+ T cells and epithelial cells are activated and release pro-inflammatory mediators, oxidants, cytokines and neutophilic chemotactic factors, IL-8, GROα, ENA-78 and leukotrienes. IL-8, GROα and ENA-78 are selective chemoattractants for neutrophils. In human neutrophils IL-8 binds two distinct receptors with similar affinity, CXCR1 and CXCR2. Closely related chemokines including GROα, β, γ, NAP-2 and ENA-78 bind only to CXCR2. Inhibiting neutrophil recruitment is therefore a recognised therapeutic strategy for treating several lung diseases. Blocking the binding of IL-8, GROα and ENA-78 to the chemokine receptor CXCR2 can provide beneficial effects in patients with COPD by suppressing the infiltration and activation of key inflammatory cells, thereby reducing subsequent tissue damage, mucus secretion, airflow obstruction and disease progression.

The IL-8 and GROα chemokine inhibitory properties of agents of the invention can be demonstrated in the following assays:

### Receptor Binding Assay

[¹²⁵I] IL-8 (human recombinant) was obtained from Amersham Pharmacia Biotech., with specific activity 2000 Ci/mmol. All other chemicals were of analytical grade. Human recombinant CXCR2 receptor expressed in Chinese hamster ovary cells (CHO-K1) was purchased from Euroscreen. The Chinese hamster ovary membranes were prepared according to protocol supplied by Euroscreen. Membrane protein concentration was determined using a Bio-Rad protein assay. Assays were performed in a 96-well micro plate format according the method described in White, et al., J Biol Chem., 1998, 273, 10095). Each reaction mixture contained 0.05 mg/ml CXCR2 membrane protein in 20 mM Bis-Tris-propane, pH 8.0, containing 1.2 mM MgSO₄, 0.1 mM EDTA, 25 mM NaCl and 0.03% CHAPS. In addition, compound of interest was added which had been pre-dissolved in dimethylsulphoxide (DMSO) so as to reach a final concentration of between 10 µM and 0.0005 µM (final concentration of DMSO 2 % (v/v)). Binding was initiated by addition of 0.02 nM ¹²⁵I-IL-8. After 2 hours at room temperature the plate was harvested using a Brandell™ 96-well harvester onto glass fibre filter plate (GF/c) blocked with 1% polyethyleneimine + 0.5% BSA and washed 3 times with 25 mM NaCl, 10 mM TrisHCl, 1 mM MgSO₄, 0.5 mM EDTA, 0.03% CHAPS, pH 7.4. The filter was dried at 50°C overnight. Backseal was then applied to the plate and 50 µl of liquid scintillation fluid added. The counts were then measured on the Packard Topcount™ scintillation counter.

### [³⁵S]-GTPγS binding assay for human CXCR2 receptor using SPA technology

[³⁵S]-GTPγS (with specific activity 1082 Ci/mmol) and wheat germ agglutinin poly vinyl toluene scintillation proximity beads were purchased from Amersham Pharmacia Biotech. The Chinese hamster ovary cell (CHO-K1) membranes expressing human CXCR2 receptors were purchased from Biosignal Packard Inc. All other chemicals were of analytical grade. White non-binding surface 96 well Optiplate™ microplates were obtained from Packard. Recombinant human IL-8 was synthesised, cloned and expressed in *Escherichia coli* as described previously (Lindley I, et al., Proc. Natl. Acad. Sci., 1988, 85(23):9199). The assay was performed in duplicate in 96 well Optiplate™ microplate in a final volume of 250 µl per well. Compounds were diluted in DMSO (0.5% final concentration) and incubated in 20 mM HEPES buffer pH 7.4 containing 10 mM MgCl₂, 100 mM NaCl, 1 mM EDTA plus 100 nM IL-8, 50 µM GDP and 500 pM [³⁵S]GTPγS per well. SPA beads (1 mg/well final concentration) were pre-mixed with the membranes (10 µg/well final concentration) in assay buffer: 20 mM HEPES buffer pH 7.4 containing 10 mM MgCl₂, 100 mM NaCl, 1 mM EDTA. The bead membrane mixture was then added to each well, plates were sealed and incubated at room temperature for 60 minutes. After which time the plate was centrifuged and immediately read on Packard TopCount™ scintillation counter, program [³⁵S dpm] for 1 min/well. Data was expressed as the % response to 100 nM IL-8 minus basal.

### Chemotaxis Assay

The *in vitro* inhibitory properties of these compounds are determined in the neutrophil chemotaxis assay. Assays were performed in a 96-well plate format according to previously published method (Frevert C W, et al., J Immunolog. Methods, 1998, 213, 41). 96-well chemotaxis chambers 5 µm were obtained from Neuro Probe, all cell buffers were obtained from Invitrogen Paisley, UK, dextran -T500 and Ficoll-Paque Plus™ density gradient centrifugation media were purchased from Pharmacia Biotech Buckinghamshire, UK. Calcein-AM dye was obtained from Molecular Probes. Neutrophils were isolated as previously described (Haslett, C., et al. Am J Path., 1985, 119:101). Citrated whole blood was mixed with 4% (w/v) dextran-T500 and allowed to stand on ice for 30 minutes to remove erythrocytes. Granulocytes (PMN) were separated from peripheral blood mononuclear cells by layering 15 ml of cell suspension onto 15 ml Ficoll-Paque PLUS density gradient and centrifuged at 250 xg for 25 minutes. Following centrifugation any erythrocytes contamination of PMN pellet was removed by hypotonic shock lysis using 10 ml ice-cold endotoxin-free sterile water for 50 seconds and neutralised with 10 ml of cold 2x phosphate buffered saline. Isolated neutrophils (1 x10⁷) were then labelled with the fluorochrome calcein-AM (5 µg) in a total volume of 1 ml and incubated for 30 minutes at 37°C. The labelled cells were washed with RPMI without phenol red + 0.1% bovine serum albumin, prior to use the cells were counted and adjusted to a final concentration of 5 x 10⁶ cells /ml. The labelled neutrophils were then mixed with test compounds (0.001 - 1000 nM) diluted in DMSO (0.1% final concentration) and incubated for 10 minutes at room temperature. The chemoattractants (29 µl) are placed in the bottom chamber of a 96-well chemotaxis chamber at a concentration between (0.1 - 5 nM). The polycarbonate filter (5 µm) was overlaid on the plate, and the cells (25 µl) were loaded on the top filter. The cells were allowed to migrate for 90 minutes at 37° C in a humidified incubator with 5% CO₂. At the end of the incubation period, migrated cells were quantified using a multi-well fluorescent plate reader (Fluroskan II™, Labsystems) at 485 nm excitation and 538 nm emission. Each compound is tested in quadruplet using 4 different donors. Positive control cells, i.e. cells that have not been treated with compound, are added to the bottom well. These represent the maximum chemotactic response of the cells. Negative control cells, i.e. those that have not been stimulated by a chemoattractant, are added to the bottom chamber. The difference between the positive control and negative control represents the chemotactic activity of the cells.

The compounds of the Examples herein below generally have IC₅₀ values below 2 µM in the above assay. For instance, the compounds of Examples 2 and 3 have IC₅₀ values of 0.322 and 0.137 µM respectively.

Having regard to their inhibition of binding of CXCR2, agents of the invention are useful in the treatment of conditions mediated by CXCR2, for example inflammatory or allergic conditions, particularly chronic obstructive pulmonary airways or lung disease (COPD, COAD or COLD), including chronic bronchitis or dyspnea associated therewith, emphysema, and severe asthma. Treatment in accordance with the invention may be symptomatic or prophylactic.

Prophylactic efficacy in the treatment of chronic bronchitis or COPD will be evidenced by reduced frequency or severity, will provide symptomatic relief and reduce disease progression, improvement in lung function. It may further be evidenced by reduced requirement for other, symptomatic therapy, i.e. therapy for or intended to restrict or abort symptomatic attack when it occurs, for example anti-inflammatory (e.g. corticosteroid) or bronchodilatory.

Other inflammatory or obstructive airways diseases and conditions to which the present invention is applicable include acute lung injury (ALI), acute/adult respiratory distress syndrome (ARDS), idiopathic pulmonary fibrosis, fibroid lung, airway hyperresponsiveness, dyspnea, pulmonary fibrosis, allergic airway inflammation, small airway disease, lung carcinoma, acute chest syndrome in patients with sickle cell disease and pulmonary hypertension, as well as exacerbation of airways hyperreactivity consequent to other drug therapy, in particular other inhaled drug therapy. The invention is also applicable to the treatment of bronchitis of whatever type or genesis including, e.g., acute, arachidic, catarrhal, croupus, chronic or phthinoid bronchitis. Further inflammatory or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tabacosis and byssinosis.

Agents of the invention are also useful for treating respiratory viral infections, which exacerbate underlying chronic conditions such as asthma, chronic bronchitis, COPD, otitis media, and sinusitis. The respiratory viral infection treated may be associated with secondary bacterial infection, such as otitis media, sinusitis or pneumonia.

Agents of the invention are also useful in the treatment of inflammatory conditions of the skin, for example psoriasis, atopic dermatitis, lupus erythematosus, and other inflammatory or allergic conditions of the skin.

Agents of the invention may also be used for the treatment of other diseases or conditions, in particular diseases or conditions having an inflammatory component, for example, diseases affecting the nose including allergic rhinitis, e.g. atrophic, chronic, or seasonal rhinitis, inflammatory conditions of the gastrointestinal tract, for example inflammatory bowel disease such as ulcerative colitis and Crohn's disease, diseases of the bone and joints including rheumatoid arthritis, psoriatic arthritis, and other diseases such as atherosclerosis, multiple sclerosis, and acute and chronic allograft rejection, e.g. following transplantation of heart, kidney, liver, lung or bone marrow.

Agents of the invention are also useful in the treatment of endotoxic shock, glomerulonephritis, cerebral and cardiac ischemia, Alzheimer's disease, cystic fibrosis, virus infections and the exacerbations associated with them, acquired immune deficiency syndrome (AIDS), multiple sclerosis (MS), *Helicobacter pylori* associated gastritis, and cancers, particularly the growth of ovarian cancer.

Agents of the invention are also useful for treating symptoms caused by viral infection in a human which is caused by the human rhinovirus, other enterovirus, coronavirus, herpes viruses, influenza virus, parainfluenza virus, respiratory syncytial virus or an adenovirus.

The effectiveness of an agent of the invention in inhibiting inflammatory conditions, for example in inflammatory airways diseases, may be demonstrated in an animal model, e.g. mouse, rat or rabbit model, of airway inflammation or other inflammatory conditions, for example as described by Wada et al, J. Exp. Med (1994) 180:1135-40; Sekido et al, Nature (1993) 365:654-57; Modelska et al., Am. J. Respir. Crit. Care. Med (1999) 160:1450-56; and Laffon et al (1999) Am. J. Respir. Crit. Care Med. 160:1443-49.

The agents of the invention are also useful as co-therapeutic agents for use in combination with other drug substances such as anti-inflammatory, bronchodilatory, antihistamine or anti-tussive drug substances, particularly in the treatment of obstructive or inflammatory airways diseases such as those mentioned hereinbefore, for example as potentiators of therapeutic activity of such drugs or as a means of reducing required dosaging or potential side effects of such drugs. An agent of the invention may be mixed with the other drug substance in a fixed pharmaceutical composition or it may be administered separately, before, simultaneously with or after the other drug substance. Accordingly the invention includes a combination of an agent of the invention as hereinbefore described with an anti-inflammatory, bronchodilatory, antihistamine or anti-tussive drug substance, said agent of the invention and said drug substance being in the same or different pharmaceutical composition.

Suitable anti-inflammatory drugs include steroids, in particular glucocorticosteroids such as budesonide, beclamethasone dipropionate, fluticasone propionate, ciclesonide or mometasone furoate, or steroids described in WO 02/88167, WO 02/12266, WO 02/100879, WO 02/00679 (especially those of Examples 3, 11, 14, 17, 19, 26, 34, 37, 39, 51, 60, 67, 72, 73, *90, 99* and 101), WO 03/35668, WO 03/48181, WO 03/62259, WO 03/64445, WO 03/72592, WO 04/39827 and WO 04/66920; non-steroidal glucocorticoid receptor agonists, such as those described in DE 10261874, WO 00/00531, WO 02/10143, WO 03/82280, WO 03/82787, WO 03/86294, WO 03/104195, WO 03/101932, WO 04/05229, WO 04/18429, WO 04/19935 and WO 04/26248; LTD4 antagonists such as montelukast and zafirlukast; PDE4 inhibitors such cilomilast (Ariflo® GlaxoSmithKline), Roflumilast (Byk Gulden),V-11294A (Napp), BAY19-8004 (Bayer), SCH-351591 (Schering-Plough), Arofylline (Almirall Prodesfarma), PD189659 / PD168787 (Parke-Davis), AWD-12-281 (Asta Medica), CDC-801 (Celgene), SelCID(TM) CC-10004 (Celgene), VM554/UM565 (Vernalis), T-440 (Tanabe), KW-4490 (Kyowa Hakko Kogyo), and those disclosed in WO 92/19594, WO 93/19749, WO 93/19750, WO 93/19751, WO 98/18796, WO 99/16766, WO 01/13953, WO 03/104204, WO 03/104205, WO 03/39544, WO 041000814, WO 04/000839, WO 04/005258, WO 04/018450, WO 04/018451, WO 04/018457, WO 04/018465, WO 04/018431, WO 04/018449, WO 04/018450, WO 04/018451, WO 04/018457, WO 04/018465, WO 04/019944, WO 04/019945, WO 04/045607 and WO 04/037805; A_{2A} agonists such as those described in EP 1052264, EP 1241176, EP 409595A2, WO 94/17090, WO 96/02543, WO 96/02553, WO 98/28319, WO 99/24449, WO 99/24450, WO 99/24451, WO 99/38877, WO 99/41267, WO 99/67263, WO 99/67264, WO 99/67265, WO 99/67266, WO 00/234-57, WO 00/77018, WO 00/78774, WO 01/23399, WO 01/27130, WO 01/27131, WO 01/60835, WO 01/94368, WO 02/00676, WO 02/22630, WO 02/96462, and WO 03/086408; and A_{2B} antagonists such as those described in WO 02/42298.

Suitable bronchodilatory drugs include anticholinergic or antimuscarinic agents, in particular ipratropium bromide, oxitropium bromide, tiotropium salts and CHF 4226 (Chiesi), and glycopyrrolate, but also those described in EP 424021, US 3714357, US 5171744, WO 01/04118, WO 02/00652, WO 02/51841, WO 02/53564, WO 03/00840, WO 03/33495, WO 03/53966, WO 03/87094, WO 04/018422 and WO 04/05285; and beta-2 adrenoceptor agonists such as albuterol (salbutamol), metaproterenol, terbutaline, salmeterol fenoterol, procaterol, and especially, formoterol, carmoterol and pharmaceutically acceptable salts thereof, and compounds (in free or salt or solvate form) of formula I of WO 00/75114, which document is incorporated herein by reference, preferably compounds of the Examples thereof, especially a compound of formula 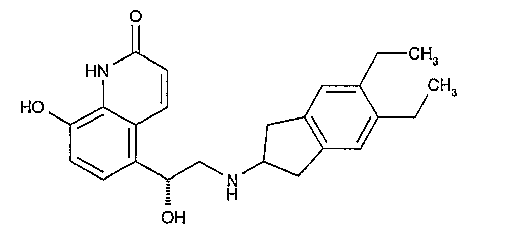 and pharmaceutically acceptable salts thereof, as well as compounds (in free or salt or solvate form) of formula I of WO 04/16601, and also compounds of EP 1440966, JP 05025045, WO 93/18007, WO 99/64035, US 2002/0055651, WO 01/42193, WO 01/83462, WO 02/66422, WO 02/ 70490, WO 02/76933, WO 03/24439, WO 03/42160, WO 03/42164, WO 03/72539, WO 03/91204, WO 03/99764, WO 04/16578, WO 04/22547, WO 04/32921, WO 04/33412, WO 04/37768, WO 04/37773, WO 04/37807, WO 04/39762, WO 04/39766, WO 04/45618 WO 04/46083 and WO 04/80964.

Such antihistamine drug substances include cetirizine hydrochloride, acetaminophen, clemastine fumarate, promethazine, loratidine, desloratidine, diphenhydramine and fexofenadine hydrochloride.

Combinations of agents of the invention and anticholinergic or antimuscarinic agents, steroids, beta-2 agonists, PDE4 inhibitors, dopamine receptor agonists, LTD4 antagonists or LTB4 antagonists may also be used. Other useful combinations of agents of the invention with anti-inflammatory drugs are those with other antagonists of chemokine receptors, e.g. CCR-1, CCR-3, CCR-4, CCR-5, CCR-6, CCR-7, CCR-8, CCR-9 and CCR10, CXCR1, CXCR2, CXCR3, CXCR4, CXCR5, particularly CCR-5 antagonists such as Schering-Plough antagonists SC-351125, SCH-55700 and SCH-D, Takeda antagonists such as N-[[4-[[[6,7-dihydro-2-(4-methylphenyl)-5H-benzocyclohepten-8-yl]carbonyl]amino]phenyl]-methyl]-tetrahydro-N,N-dimethyl-2H-pyran-4-aminium chloride (TAK-770), CCR-5 antagonists described in US 6166037 (particularly claims 18 and 19), WO 0066558 (particularly claim 8), and WO 0066559 (particularly claim 9).

In accordance with the foregoing, the invention also provides a method for the treatment of a condition mediated by CXCR2, for example an inflammatory or allergic condition, particularly an inflammatory or obstructive airways disease, which comprises administering to a subject, particularly a human subject, in need thereof an effective amount of a compound of formula I in a free or pharmaceutically acceptable salt form as hereinbefore described. In another aspect the invention provides the use of a compound of formula I, in free or pharmaceutically acceptable salt form, as hereinbefore described for the manufacture of a medicament for the treatment of a condition mediated by CXCR2, for example an inflammatory or allergic condition, particularly an inflammatory or obstructive airways disease.

The agents of the invention may be administered by any appropriate route, e.g. orally, for example in the form of a tablet or capsule; parenterally, for example intravenously; by inhalation, for example in the treatment of inflammatory or obstructive airways disease; intranasally, for example in the treatment of allergic rhinitis; topically to the skin, for example in the treatment of atopic dermatitis; or rectally, for example in the treatment of inflammatory bowel disease.

In a further aspect, the invention also provides a pharmaceutical composition comprising as active ingredient a compound of formula I in free or pharmaceutically acceptable salt form, optionally together with a pharmaceutically acceptable diluent or carrier therefor. The composition may contain a co-therapeutic agent such as an anti-inflammatory bronchodilatory or antihistamine drug as hereinbefore described. Such compositions may be prepared using conventional diluents or excipients and techniques known in the galenic art. Thus oral dosage forms may include tablets and capsules. Formulations for topical administration may take the form of creams, ointments, gels or transdermal delivery systems, e.g. patches. Compositions for inhalation may comprise aerosol or other atomizable formulations or dry powder formulations.

When the composition comprises an aerosol formulation, it preferably contains, for example, a hydro-fluoro-alkane (HFA) propellant such as HFA134a or HFA227 or a mixture of these, and may contain one or more co-solvents known in the art such as ethanol (up to 20% by weight), and/or one or more surfactants such as oleic acid or sorbitan trioleate, and/or one or more bulking agents such as lactose. When the composition comprises a dry powder formulation, it preferably contains, for example, the compound of formula I having a particle diameter up to 10 microns, optionally together with a diluent or carrier, such as lactose, of the desired particle size distribution and a compound that helps to protect against product performance deterioration due to moisture, e.g. magnesium stearate. When the composition comprises a nebulised formulation, it preferably contains, for example, the compound of formula I either dissolved, or suspended, in a vehicle containing water, a co-solvent such as ethanol or propylene glycol and a stabiliser, which may be a surfactant.

The invention includes (A) an agent of the invention in inhalable form, e.g. in an aerosol or other atomisable composition or in inhalable particulate, e.g. micronised form, (B) an inhalable medicament comprising an agent of the invention in inhalable form; (C) a pharmaceutical product comprising such an agent of the invention in inhalable form in association with an inhalation device; and (D) an inhalation device containing an agent of the invention in inhalable form.

Dosages of agents of the invention employed in practising the present invention will of course vary depending, for example, on the particular condition to be treated, the effect desired and the mode of administration. In general, suitable daily dosages for administration by inhalation are of the order of 0.01 to 1 mg/kg per day while for oral administration suitable daily doses are of the order of 0.005 to 100 mg/kg of total body weight. The daily? parenteral dosage regimen about 0.001 to about 80 mg/kg of total body weight. The daily topical dosage regimen will preferably be from 0.1 mg to 150 mg, administered one to four, preferably two or three times daily.

The invention is illustrated by the following Examples.

### EXAMPLES

Especially preferred compounds of formula I are also compounds of formula V 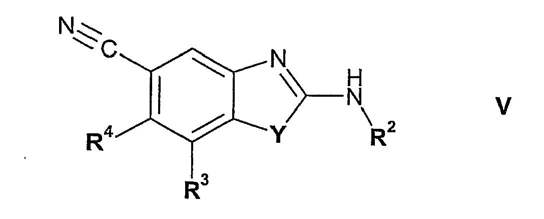 wherein Y, R², R³, and R⁴ are as shown in Table1 below, the methods of preparation being described hereinafter. All compounds are in the free form. The table also shows characterising mass spectrometry data.

**TABLE 1**

| **Ex.** | **R²** | **Y** | **R³** | **R⁴** | **MS [AP⁺]** |
|---|---|---|---|---|---|
| 1 | 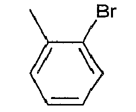 | N | -OH | -H | 329 |
| 2 | 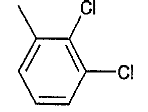 | N | -OH | -H | 319 |
| 3 | 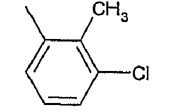 | N | -OH | -H | 299 |
| 4 | 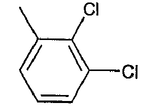 | N | -COOH | -H | 346.89 |
| 5 | 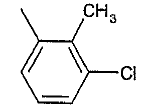 | N | -COOH | -H | - |
| 6 | 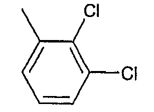 | N | -OH | -SO₂-NH₂ | - |
| 7 | 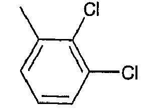 | N | 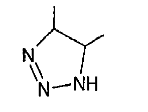 | | 344 |
| 8 | 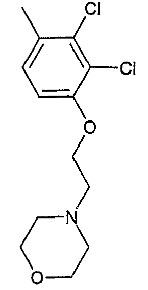 | N | -OH | -H | - |
| 9 | 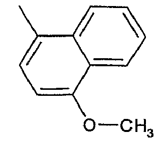 | N | -OH | -H | - |
| 10 | 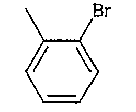 | O | -OH | -H | - |
| 11 | 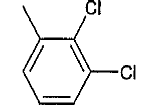 | O | -OH | -H | - |
| 12 | 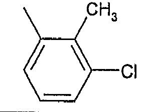 | O | -OH | -H | - |
| 13 | 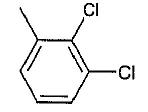 | O | -COOH | -H | - |
| 14 | 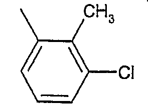 | O | -COOH | -H | - |

Especially preferred compounds of formula I are shown in Table 2 below, the methods of preparation being described hereinafter. All compounds are in the free form, except for Example 30 which is a trifluoroacetic acid salt. The table also shows characterising mass spectrometry data.

**TABLE 2**

| **Ex.** | **Compound** | **MS [AP⁺]** |
|---|---|---|
| 15 | 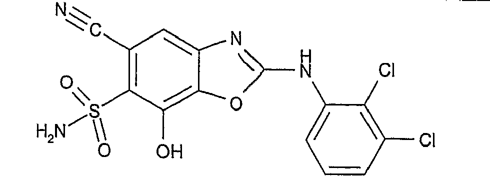 | - |
| 16 | 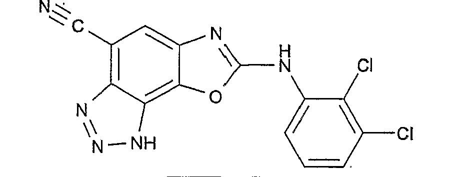 | - |
| 17 | 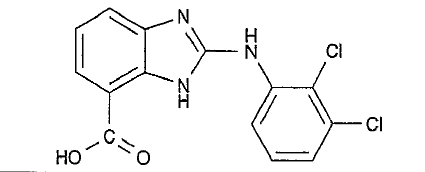 | 322 |
| 18 | 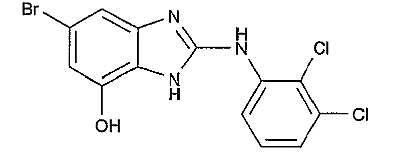 | 373 |
| 19 | 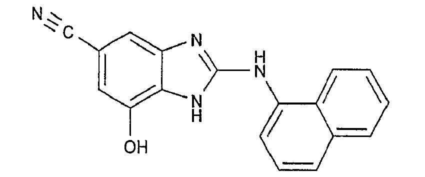 | 300 |
| 20 | 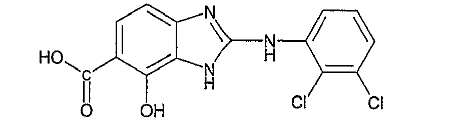 | 344 |
| 21 | 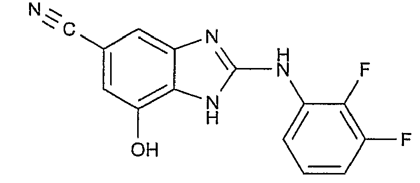 | 286 |
| 22 | 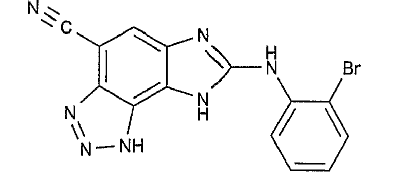 | 354 |
| 23 | 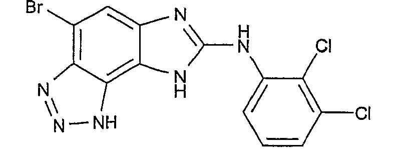 | 398 |
| 24 | 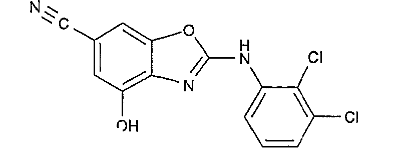 | - |
| 25 | 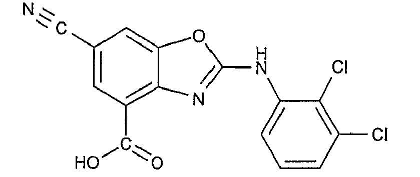 | - |
| 26 | 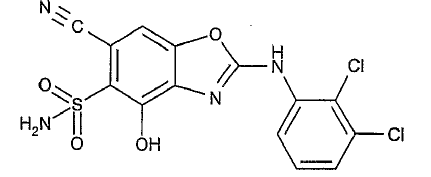 | - |
| 27 | 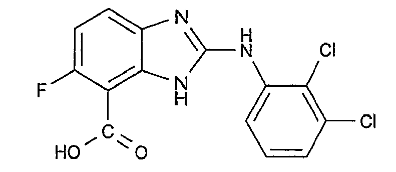 | 340 |
| 28 | 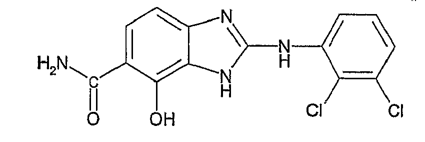 | 337 |
| 29 | 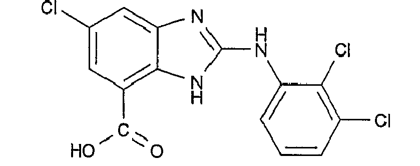 | 356 |
| 30 | 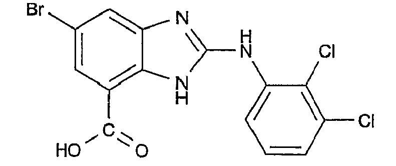 | 401 |
| 31 | 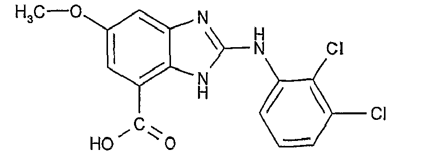 | 352 |
| 32 | 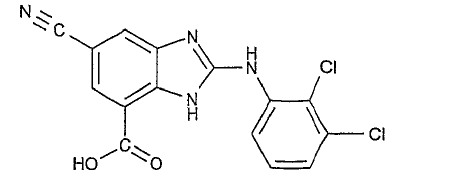 | 347 |

### Preparation of starting compounds

### 2-Methoxy-6-nitrophenylamine

Tetrabutylammonium iodide (400 mg), sodium hydroxide (4 g in water, 40 ml) and methyl iodide (3.4 ml) are added to a solution of 2-amino-3-nitrophenol (4 g) in tetrahydrofuran (THF, 80 ml) and the mixture stirred at room temperature overnight. The mixture is then concentrated *in vacuo* and brine (200 ml) and ethyl acetate (200 ml) added. The aqueous layer is re-extracted with ethyl acetate and the combined organic layers are washed with saturated aqueous sodium bicarbonate then brine and dried (MgSO₄). Filtration followed by concentration *in vacuo* yields the title compound as a brown solid. MS (AP+) 168

### 4-Bromo-2-methoxy-6-nitro-phenylamine

Sodium acetate (3.37 g) and bromine (1.3 ml) are added to a solution of 2-methoxy-6-nitrophenylamine (4.16 g) in acetic acid (50 ml). After 20 minutes. the resultant precipitate is filtered off, washed with water and dried *in vacuo* to give the title compound as an orange solid. M.p. 137-8°C. MS (AP+) 247.

### 4-Amino-3-methoxy-5-nitrobenzonitrile

Copper (I) cyanide (2.22 g) is added to a solution of 4-bromo-2-methoxy-6-nitro-phenylamine (2.04 g) in DMF/Pyridine (7 ml/1 ml) and the mixture heated at 160°C for 6 hours. On cooling, the mixture is diluted with ethyl acetate and passed through a pad of Celite™ filter material. The filtrate is washed with water and the aqueous layer re-extracted with ethyl acetate. The combined organic extracts are washed with dilute brine then saturated brine and dried (MgSO₄). Filtration and concentration *in vacuo* followed by column chromatography eluting with 30-50% ethyl acetate/hexane yields the title compound as a yellow solid. MS (AP+) 193.

### 3,4-Diamino-5-methoxy-benzonitrile

Concentrated HCl (7 ml) is added dropwise over 15 minutes to a mixture of 4-amino-3-methoxy-5-nitrobenzonitrile (1.05 g) and tin (II) chloride powder (2.13 g) in ethanol (40 ml). The mixture is warmed to 40°C to elicit a colour change from orange to green to colourless and then stirred at room temperature for 1.5 hour. The mixture is filtered over Celite™ filter material, washing with methanol and the filtrate concentrated *in vacuo.* The aqueous layer is made basic with saturated aqueous sodium bicarbonate and dichloromethane (DCM) added. The mixture is then filtered over Celite™ filter material and the aqueous layer re-extracted with DCM. The combined organic extracts are washed with brine, dried (MgSO₄), filtered and concentrated *in vacuo* to give the title compound as a pale yellow solid. MS (AP+) 163

### Preparation of final compounds

### Example 1

### 2-(2-Bromophenylamino)-7-hydroxy-1H-benzoimidazole-5-carbonitrile

(a) 2-(2-Bromophenylamino)-7-methoxy-1H-benzoimidazole-5-carbonitrile:
   2-Bromophenylisothiocyanate (487 µl) is added to a solution of 3,4-diamino-5-methoxy-benzonitrile (590 mg) in ethanol (35 ml). The mixture is stirred at room temperature for 19 hours after which a precipitate forms. DCM (30 ml) is added to the mixture, followed by 1-(-3-dimethylamino-propyl)-3-ethylcarbodiimide hydrochloride (EDCI, 832 mg) and the mixture is heated to 80°C for 6 hours. After this, further EDCI (300 mg) is added and heating continued for 1 hour. After cooling, the reaction mixture is concentrated *in vacuo* and the residue partitioned between DCM and water. The organic phase is washed with brine then dried (MgSO₄), filtered and concentrated. Purification is achieved *via* column chromatography, eluting with 25-30% ethyl acetate:hexane to yield the desired product. MS (AP+) 343
(b) 2-(2-Bromophenylamino)-7-hydroxy-1H-benzoimidazole-5-carbonitrile:
   2-(2-Bromophenylamino)-7-methoxy-1H-benzoimidazole-5-carbonitrile (82 mg) is dissolved in 2,4,6-collidine (4 ml) with lithium iodide (64 mg) and the mixture heated at 180°C for 6 hours. during which a precipitate forms. The precipitate is filtered and washed with water to yield the title compound. MS (AP+) 329.

### Example 2

### 2-(2,3-Dichloro-phenylamino)-7-hydroxy-1H-benzoimidazole-5-carbonitrile

(a) 2-(2,3-Dichloro-phenylamino)-7-methoxy-1H-benzoimidazole-5-carbonitrile:
   A solution of 3,4-diamino-5-methoxy-benzonitrile is dissolved in ethanol (30 ml) and treated with 2,3-dichlorophenyl isothiocyanate (1.24 g). The suspension is stirred at room temperature overnight. The resulting solid is removed by filtration, triturated with ether and dried to give the thiourea intermediate as a beige solid (1.56 g). A mixture of this intermediate (1.47 g) and EDCI (0.92 g) is refluxed for 3 hours in ethanol (25 ml). The reaction mixture is concentrated to a solid and triturated with 50% water/methanol (100 ml) to give 2-(2,3-dichloro-phenyl-amino)-7-methoxy-1H-benzoimidazole-5-carbonitrile as a beige solid after filtration and drying. M.P. 291 °C dec. M.S. (AP+) 333.
(b) 2-(2,3-Dichloro-phenylamino)-7-hydroxy-1H-benzoimidazole-5-carbonitrile:
   2-(2,3-dichloro-phenylamino)-7-methoxy-1H-benzoimidazole-5-carbonitrile (492 mg) is dissolved in 2,4,6-collidine (10 ml) and heated with lithium iodide (176 mg) at 180°C for 3 hours. The reaction mixture is cooled, treated with water (15 ml) and washed twice with ethyl acetate (2 x 15 ml). The ethyl acetate extracts are combined, washed with water, dried (MgSO₄) and concentrated to a brown oil. Chromatography in 30% ethyl acetate/hexane gives the product as a beige solid. M.p. 299 °C dec. M.S. (AP+) 319

### Examples 3 to 14

The compounds of Examples 3 to 14, are prepared using procedures analogous to those used in Example 1, using appropriate starting materials.

### Examples 15 to 26

These compounds are prepared using procedures analogous to those used in Example 1, using appropriate starting materials.

### Example 27

### 2-(2,3-Dichloro-phenylamino)-5-fluoro-3H-benzoimidazole-4-carboxylic acid

(a) 2,3-Diamino-6-fluoro-benzoic acid ethyl ester:
   Commercially available 2-amino-6-fluoro-3-nitrobenzoic acid ethyl ester (1 g) is stirred at 60°C for 6 hours in ethyl acetate (40 mL) with tin (II) chloride dihydrate (2.5 g). After cooling to room temperature overnight, the reaction mixture is heated to 70° C for a further 4 hours. The solvent is then removed under vacuum and, with cooling over an ice bath, the products are partitioned between diethylether and 4M sodium hydroxide (ca. 60 mL). The ether layer is washed with a little ice cold water, then brine. Concentration of the organic layer yields the product as a dark brown gum.
(b) 2-(2,3-Dichloro-phenylamino)-5-fluoro-3H-benzoimidazole-4-carboxylic acid ethyl ester:
   To the crude 2,3-diamino-6-fluorobenzoic acid ethyl ester (600 mg) in ethanol (15 mL) is added 2,3-dichloroisothiocyanate (620 mg), and the mixture stirred at 40°C for 2 hours. N,N'-carbonyldiimidazole (300 mg) is then added to the reaction mixture, and stirring continued for 3hours at 50° C. The mixture is then left to stand overnight at room temperature. The reaction mixture is cooled over an ice bath and diethyl ether (ca.200 mL) added, with stirring until cold (<10° C). To the ether solution is added 4M sodium hydroxide (200 mL) and the mixture stirred in the ice bath until dissolution of the solid. This ether layer is then washed with a little ice-cold water and then brine. The crude product is absorbed onto silica and purified by flash column chromatography, eluting with 10:1 isohexane:ethyl acetate. The named product is obtained as a yellow solid.
(c) 2-(2,3-Dichloro-phenylamino)-5-fluoro-3H-benzoimidazole-4-carboxylic acid:
   To a stirring solution of 2-(2,3-dichlorophenylamino)-5-fluoro-3H-benzoimidazole-4-carboxylic acid ethyl ester (280 mg) in ethanol (30 mL) at 40° C is added 2M sodium hydroxide (3 mL). After 1 hour at 40° C half of the solvent is removed *in vacuo* and the mixture is then maintained at 50° C overnight. Half of the remaining solvent is removed *in vacuo* and 1M HCl is added until pH4 is attained. The resulting precipitate is stirred for 15 minutes and then filtered, washing with water. The product is dried *in vacuo* at 50° C to yield the title compound as a light brown powder.

### Example 28

### 2-(2,3-Dichloro-phenylamino)-4-hydroxy-3H-benzoimidazole-5-carboxylic acid amide

(a) 4-Acetylamino-5-chloro-2-methoxy-3-nitrobenzoic acid methyl ester:
   Fuming nitric acid (33 cm³) is added drop wise over 20 minutes to 4-acetylamino-5-chloro-2-methoxy-benzoic acid methyl ester (16.0 g) at room temperature. After stirring for an additional 10 minutes at that temperature, the mixture is poured into ice water and EtOAc (100 cm³) is added. After separating the organic layer, the aqueous layer is extracted with EtOAc (100 cm³). The combined organic layers are washed with brine (100 cm³), dried (Na₂SO₄) and evaporated under reduced pressure to give the crude product as an orange solid.
(b) 4-Amino-5-chloro-2-methoxy-3-nitrobenzoic acid methyl ester:
   Concentrated sulfuric acid (8 cm³) is added drop wise to the nitro-compound (12.5g) in MeOH (140 cm³) and the resulting mixture is heated to reflux for 9 h. After allowing the reaction to cool to room temperature, the mixture is poured into ice (1200 cm³) and the precipitate formed collected by filtration. EtOAc (100 cm³) is added and the organic layer is washed with brine (100 cm³) and water (100 cm³) , dried (Na₂SO₄) and evaporated under reduced pressure to give the amine (9.6 g, 89%) as a brown oil.
(c) 3,4-Diamino-2-methoxy-3-nitrobenzoic acid methyl ester:
   Pd-C (10 wt%, 4.5 g) is added in one portion to a stirred solution of the amine (5 g) in methanol (250 cm³) at room temperature. Then, triethylamine (30 cm³) is added and the resulting mixture is transferred to a parr apparatus. After stirring for 8 hours, the reaction mixture is filtered through Celite® filter material and washed with methanol (100 cm³). Evaporation under reduced pressure gives the crude product containing residual triethylamine. Purification by column chromatography on silica with *iso*-hexane-EtOAc (1:4) as eluent gives the diamine as a brown solid.
(d) 2-(2,3-Dichloro-phenylamino)-4-methoxy-3H-benzoimidazole-5-carboxylic acid methyl ester:
   2,3-Dichloroisothiocyanate (1.0 g) is added drop wise to the diamine (1.0 g) in EtOH (20 cm³) at room temperature under argon. After stirring for 1 hour, *N-*(3-Dimethylaminopropyl)-*N*'-ethylcarbodiimide hydrochloride (1.2 g) is added and the resulting mixture is heated using microwave radiation at 120 °C. After 12 minutes, the reaction mixture is allowed to cool to room temperature and the resulting solid collected by filtration to give the benzimidazole (1.5 g, 78%) as an off-white solid.
(e) 2-(2,3-Dichloro-phenylamino)-4-hydroxy-3H-benzoimidazole-5-carboxylic acid:
   Concentrated hydrobromic acid (4 cm³) is added drop wise to the benzimidazole (400 mg) in AcOH (4 cm³) and the resulting mixture is heated at reflux for 2 hours. After allowing the reaction mixture to cool to room temperature, water (10 cm³) is added and the resulting solid collected by filtration and washed with water (10 cm³) to give the hydroxy-acid (330 mg, 89%) as a white solid.
(f) 2-(2,3-Dichloro-phenylamino)-4-hydroxy-3H-benzoimidazole-5-carboxylic acid amide:
   1-Hydroxy-7-azabenzotriazole (13 mg) is added in one portion to a mixture of hydroxy-acid (30 mg) and *N*-(3-Dimethylaminopropyl)-*N*'-ethylcarbodiimide hydrochloride (13 mg) in DMF (0.5 cm³) at room temperature under argon. After stirring for 1 hour, ammonium hydroxide (12 µl) is added and the resulting mixture is stirred at room temperature. After stirring for 2 hours, the reaction mixture is evaporated under reduced pressure and purified by reverse phase column chromatography on Isolute SPE solid phase extraction sorbent with MeCN (0.1%TFA)-H₂O (0.1% TFA) as gradient eluent. The combined column fractions are partially evaporated under reduced pressure to remove excess MeCN. Then, a mixture of EtOAc (10 cm³) and saturated NaHCO₃ (10 cm³) is added. The layers are separated and the organic layer is dried (Na₂SO₄) and evaporated under reduced pressure to give the carboxamide as a white-orange solid.

### Example 29

### 6-Chloro-2-(2,3-dichloro-phenylamino)-3H-benzoimidazole-4-carboxylic acid

(a) 3-Amino-5-chloro-2-nitrobenzoic acid:
   1,1,1-trimethylhydrazinium iodide (12.0 g) is added in one portion to 5-chloro-2-nitro-benzoic acid (10.0 g) in dry DMSO (500 cm³) at room temperature under argon. Then, sodium *tert-*pentoxide (16.3 g) is added portion-wise and the resulting deep red solution is stirred at room temperature. After stirring for 16 hours, the reaction mixture is cooled to 0° C and quenched with 1M hydrochloric acid (150cm³) to pH 3. Then, the aqueous mixture is extracted with EtOAc (3 x 200 cm³) and the combined organic extracts are dried (Na₂SO₄) and evaporated under reduced pressure to give the crude acid as a yellow viscous oil.
(b) 3-Amino-5-chloro-2-nitrobenzoic acid methyl ester:
   Methyl iodide (0.73 cm³) is added drop wise to a mixture of the crude acid (2.5 g) and K₂CO₃ (2.1 g) in DMF (17 cm³) at room temperature under argon. The resulting mixture is heated in the microwave at 60° C for 10 minutes. After being allowed to cool to room temperature, the mixture is poured carefully into a mixture of EtOAc (50 cm³) and 1 M hydrochloric acid (50 cm³). Then, the layers are separated and the organic layer is washed with saturated Na₂CO₃ (50cm³), brine (50 cm³) and water (50 cm³), dried (Na₂SO₄) and evaporated under reduced pressure to give the crude product as a brown oil. Purification by column chromatography on silica with *iso*-hexane-EtOAc (3:1) as eluent gives the ester as a yellow solid.
(c) 2,3-Diamino-5-chlorobenzoic acid methyl ester:
   1 M hydrochloric acid (0.7 cm³) is added drop wise to the ester (2.0 g) in glacial AcOH (70 cm³). Then, zinc dust (4.5 g) is added portion wise and the resulting suspension is stirred at room temperature under argon. After stirring for 1 hour, the mixture is treated with an excess of saturated potassium carbonate solution. The aqueous mixture is extracted with EtOAc (2 x 50 cm³) and the combined organic extracts are washed with brine (50 cm³) and water (50 cm³), dried (Na₂SO₄) and evaporated under reduced pressure to give the crude product as a dark brown solid. Purification by column chromatography on silica with *iso*-hexane-EtOAc (2:1) as eluent gives the diamine as a pink solid.
(d) 6-Chloro-2-(2,3-dichloro-phenylamino)-3H-benzoimidazole-4-carboxylic acid methyl ester:
   2,3-Dichloroisothiocyanate (243 mg) is added drop wise to the diamine (150 mg) in MeOH (6 cm³) at room temperature under argon. After stirring for 2 hours, methyl iodide (0.46 cm³) is added and the resulting mixture is allowed to stir at room temperature. After 16 hours, the reaction mixture is evaporated under reduced pressure to give the crude product as a brown solid. Purification by column chromatography on silica with DCM as eluent gives the benzimidazole as an off-white solid.
(e) 6-Chloro-2-(2,3-dichloro-phenylamino)-3H-benzoimidazole-4-carboxylic acid:
   2 M sodium hydroxide (0.87 cm³) is added drop wise to the benzimidazole (65 mg) in THF (2 cm³) and MeOH (0.2 cm³) and the resulting mixture is heated at 40° C for 16 hours. After allowing the reaction mixture to cool to room temperature, 1 M hydrochloric acid (2 cm³) is added to pH 2 and the resulting solid collected by filtration to give the acid as a white solid.

### Example 30

### 6-Bromo-2-(2,3-dichloro-phenylamino)-3H-benzoimidazole-4-carboxylic acid

(a) 2-Amino-5-bromo-3-nitro-benzoic acid methyl ester:
   To a stirred solution of commercially available 2-amino-3-nitro-benzoic acid methyl ester (5 g) in acetic acid (25 mL) at room temperature is added dropwise a bromine (1.286 mL) solution in acetic acid (5 mL). The resulting mixture is stirred for 45 minutes and then poured onto ice. The yellow precipitated is filtered off and dried under vacuum to afford the desired bromo compound as a yellow solid.
(b) 2,3-Diamino-5-bromo-benzoic acid methyl ester:
   To a stirred solution of 2-amino-5-bromo-3-nitro-benzoic acid methyl ester (1 g) in ethanol (25 mL) is added anhydrous tin(II) chloride(4.19 g). The reaction mixture is heated to 70° C and stirred for 16 hours. The mixture is cooled to room temperature, the solvent evaporated and the residue diluted with water, basified to pH=13 with a 40% sodium hydroxide solution and extracted with dichloromethane to afford the diamine product as a brown solid.
(c) 6-Bromo-2-(2,3-dichloro-phenylamino)-3H-benzoimidazole-4-carboxylic acid methyl ester:
   To a stirred solution of 2,3-dichlorophenylisothiocyanate (280 µL) in acetonitrile (8 mL) is added under nitrogen 2,3-Diamino-5-bromo-benzoic acid methyl ester (415 mg) at room temperature. The reaction mixture is stirred at room temperature for 0.5 hour then N-(3-dimethylaminopropyl)-N'-ethylcarbodiimide hydrochloride (474 mg) is added in one portion and the mixture is stirred at room temperature for a further 4 hours. The reaction is quenched with water and extracted with ethyl acetate. The organic layers are combined, dried over anhydrous sodium sulfate, filtrated and evaporated under vacuum. The crude product is adsorbed on isolute® solid phase extraction sorbent and purified by flash chromatography on silica gel eluting with hexane: ethyl acetate 4:1 to afford a yellow solid.
(d) 6-Bromo-2-(2,3-dichloro-phenylamino)-3H-benzoimidazole-4-carboxylic acid:
   To a stirred solution of 6-bromo-2-(2,3-dichloro-phenylamino)-3H-benzoimidazole-4-carboxylic acid methyl ester (30 mg) in tetrahydrofuran (0.8 mL) is added a solution of lithium hydroxide (29 mg) in water (0.4 mL) at room temperature. The mixture is stirred at room temperature for 16 hours. The reaction is quenched and acidified with a 1N hydrochloric acid solution to pH=4-5 and extracted with ethyl acetate. The organic layer are combined, dried over anhydrous sodium sulfate, filtrated and evaporated under vacuum. The crude product is adsorbed on isolute® solid phase extraction sorbent and purified by reverse phase chromatography on C18 silica gel eluting with a water (+0.1 % trifluoroacetic acid): acetonitrile (+0.1 % trifluoroacetic acid) gradient (80:20 to 40:60 over 30 minutes) to afford the desired carboxylic acid as a white lyophilized powder as a trifluoroacetic acid salt. ([M]⁺=401).

### Example 31

### 2-(2,3-Dichloro-phenylamino)-6-methoxy-3 H-benzoimidazole-4-carboxylic acid

(a) 3-Amino-5-methoxy-2-nitro-benzoic acid:
   To a solution of the commercially available 5-fluoro-2-nitro-benzoic acid (1 g) and O-Methylhydroxylamine (0.498 g) in dimethoxyethane (25 mL) is added dropwise a suspension of potassium tert-butanolate (4.2 g) and copper(II) acetate (96.16 mg) in DME (18 mL) over 30 minutes at room temperature. After 15 minutes at room temperature, the reaction is quenched with water. The mixture is acidified to pH=3 with 1N HCl and extracted with ethyl acetate. The organic layer is washed with brine and dried over magnesium sulfate and concentrated under vacuum. The crude oil is purified by flash chromatography on silica gel eluting with dichloromethane:methanol:acetic acid 8:2:0.1 to afford a yellow-dark solid.
(b) 3-Amino-5-methoxy-2-nitro-benzoic acid methyl ester:
   To a solution of 3-amino-5-methoxy-2-nitro-benzoic acid (0.36 g) is added concentrated sulfuric acid (1.88 mL) and the resulting solution heated up to 90° C and stirred for a further 16 hours. The reaction mixture is cooled down to room temperature, neutralized with sodium hydroxide 6N, extracted with ethyl acetate. The organic layer is dried over magnesium sulfate and concentrated under vacuum. The crude product is adsorbed on isolute® solid phase extraction sorbent and purified by flash chromatography on silica gel eluting with hexane: ethyl acetate 4:1 to afford a brown solid.
(c) 2,3-Diamino-5-methoxy-benzoic acid methyl ester:
   To a solution of 3-Amino-5-methoxy-2-nitro-benzoic acid methyl ester (100 mg) in ethanol is added tin(II) chloride (509 mg). The reaction mixture is heated to 70°C and stirred for a further 16 hours. The reaction mixture is cooled down to room temperature and evaporated under vacuum. The residue was treated with 40% aqueous sodium hydroxide to pH=12. The mixture is diluted with water and extracted with chloroform, the organic layer dried over anhydrous sodium sulfate, filtrated and evaporated under vacuum to afford a brown solid.
(d) 2-(2,3-Dichloro-phenylamino)-6-methoxy-3H-benzoimidazole-4-carboxylic acid methyl ester:
   To a stirred solution of 2,3-dichlorophenylisothiocyanate (44.4 µL) in acetonitrile (1.5 mL) is added under nitrogen 2,3-diamino-5-methoxy-benzoic acid methyl ester (50 mg) at room temperature. The reaction mixture is stirred at room temperature for 1.5 hours then N-(3-dimethylaminopropyl)-N'-ethylcarbodiimide hydrochloride (60.1 mg) is added in one portion and the mixture is stirred at room temperature for a further 4 hours. The reaction is quenched with water and extracted with ethyl acetate. The organic layer are combined, dried over anhydrous sodium sulfate, filtrated and evaporated under vacuum. The crude product is adsorbed on isolute® solid phase extraction sorbent and purified by flash chromatography on silica gel eluting with hexane: ethyl acetate 4:1 to afford a yellowish solid.
(e) 2-(2,3-Dichloro-phenylamino)-6-methoxy-3H-benzoimidazole-4-carboxylic acid:
   To a stirred solution of 2-(2,3-dichloro-phenylamino)-6-methoxy-3H-benzoimidazole-4-carboxylic acid methyl ester (45 mg) in tetrahydrofuran (1 mL) is added a solution of lithium hydroxide (52 mg) in water (0.5 mL) at room temperature. The mixture is stirred at room temperature for 24 hours. The reaction is quenched and acidified with a 1N hydrochloric acid solution to pH=4-5 and extracted with ethyl acetate. The organic layers are combined, dried over anhydrous sodium sulfate, filtrated and evaporated under vacuum to afford the desired carboxylic acid as a light yellow powder ([M]⁺=352).

### Example 32

### 6-Cyano-2-(2,3-dichloro-phenylamino)-3H-benzoimidazole-4-carboxylic acid

(a) 6-Cyano-2-(2,3-dichloro-phenylamino)-3H-benzoimidazole-4-carboxylic acid methyl ester:
   A nitrogen purged flask is loaded with 6-bromo-2-(2,3-dichloro-phenylamino)-3H-benzo-imidazole-4-carboxylic acid methyl ester (41 mg), copper(I) iodide (19 mg) and tetrakis-(triphenylphosphine)palladium (58 mg), freshly dry des-oxygenated toluene is added and the flask sealed. The suspension is then heated to 120°C for 16 hours in the dark. The reaction mixture is cooled to room temperature, diluted with ethyl acetate and 30% ammonium hydroxide. The layer separated, the organic layer washed with brine, dried over anhydrous sodium sulfate, filtrated and evaporated. The crude product is adsorbed on isolute® solid phase extraction sorbent and purified by flash chromatography on silica gel eluting with hexane: ethyl acetate: ammonium hydroxide 6:4:0.1 to afford a yellowish solid.
(b) 6-Cyano-2-(2,3-dichloro-phenylamino)-3H-benzoimidazole-4-carboxylic acid:
   To a stirred solution of crude 6-cyano-2-(2,3-dichloro-phenylamino)-3H-benzoimidazole-4-carboxylic acid methyl ester (1mg) in THF (0.4 mL) is added a solution of lithium hydroxide (15mg) in water (0.2 mL) at room temperature. The resulting solution is stirred at room temperature for a further 16 hours. The crude product is directly purified by preparative TLC using hexane:ethyl acetate: formic acid 4:6:0.1 as eluent to afford the desired product.

## Claims

1. A compound of formula I 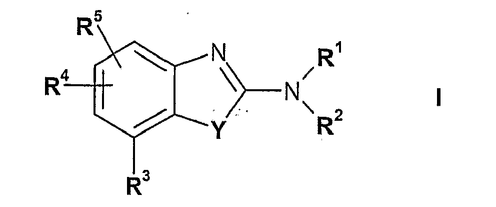 in free or salt form, where
Y is nitrogen or oxygen;
R¹ is hydrogen or C₁-C₈-alkyl;
R² is a C₃-C₁₅-carbocyclic group optionally substituted by halo, cyano, hydroxy, carboxy, nitro, aminocarbonyl, C₁-C₈-alkyl or by C₁-C₈-alkoxy optionally substituted by a 5- or 6-membered heterocyclic group containing at least one ring heteroatom selected from nitrogen, oxygen and sulphur,
or R² is C₁-C₈-alkyl or C₁-C₈-alkoxy in either case being optionally substituted by hydroxy, carboxy, -SO₂NH₂, C₁-C₈-alkylsulfonylamino or a 5- or 6-membered heterocyclic group containing at least one ring heteroatom selected from nitrogen, oxygen and sulphur,
or R² is C₁-C₈-alkylcarbonyl, C₃-C₈-cycloalkyl or a 5- or 6-membered heterocyclic group containing at least one ring heteroatom selected from nitrogen, oxygen and sulphur,
or R¹ and R² together form C₃-C₈-cycloalkyl or a 5- or 6-membered heterocyclic group containing at least one ring heteroatom selected from nitrogen, oxygen and sulphur;
R³ is hydrogen, halo, cyano, hydroxy, nitro, carboxy, aminocarbonyl, C₁-C₈-alkyl, C₁-C₈-alkenyl, C₁-C₈-alkynyl, C₁-C₈-haloalkyl, C₁-C₈-alkylthio, C₃-C₈-cycloalkyl, C₃-C₈-cycloalkyl-C₁-C₈-alkyl, carboxy-C₁-C₈-alkoxy, -CO-NR⁶R⁷, -NH-SO₂R⁸, -NH-COH or -SO₂NH₂,
and R⁴ and R⁵ are independently hydrogen, halo, cyano, hydroxy, nitro, carboxy, aminocarbonyl, C₁-C₈-alkyl, C₁-C₈-alkenyl, C₁-C₈-alkynyl, C₁-C₈-haloalkyl, C₁-C₈-alkylthio, C₁-C₈-alkoxy, C₁-C₈-haloalkoxy, C₃-C₈-cycloalkyl, C₃-C₈-cycloalkyl-C₁-C₈-alkyl, C₃-C₈-cycloalkyl-oxy, -CO-NR⁶R⁷, -NH-SO₂R⁸, -NH-COH or -SO₂NH₂,
or two of R³, R⁴ and R⁵ that are attached to adjacent ring carbon atoms together form a 5- or 6-membered heterocyclic group containing at least one ring heteroatom selected from nitrogen, oxygen and sulphur; and
R⁶, R⁷ and R⁸ are independently hydrogen or C₁-C₈-alkyl.

2. A compound according to claim 1, where
Y is nitrogen or oxygen;
R¹ is hydrogen;
R² is a C₃-C₁₅-carbocyclic group optionally substituted by halo, C₁-C₈-alkyl or by C₁-C₈-alkoxy optionally substituted by a 5- or 6-membered heterocyclic group containing at least one ring heteroatom selected from nitrogen, oxygen and sulphur; and
R³, R⁴ and R⁵ are independently hydrogen, halo, cyano, hydroxy, carboxy, -SO₂NH₂, C₁-C₈-alkoxy or -CO-NH₂,
or two of R³, R⁴ and R⁵ that are attached to adjacent ring carbon atoms together form a 5- or 6-membered heterocyclic group containing at least one ring heteroatom selected from nitrogen, oxygen and sulphur.

3. A compound according to claim 1, where
Y is nitrogen or oxygen;
R¹ is hydrogen;
R² is a C₃-C₁₀-carbocyclic group, preferably phenyl or naphthyl, optionally substituted by halo, C₁-C₄-alkyl or by C₁-C₄-alkoxy optionally substituted by a 5- or 6-membered heterocyclic group containing at least one ring heteroatom selected from nitrogen, oxygen and sulphur; and R³, R⁴ and R⁵ are independently hydrogen, halo, cyano, hydroxy, carboxy, -SO₂NH₂, C₁-C₄-alkoxy or -CO-NH₂,
or two of R³, R⁴ and R⁵ that are attached to adjacent ring carbon atoms together form a 5- or 6-membered heterocyclic group containing at least one ring heteroatom selected from nitrogen, oxygen and sulphur.

4. A compound according to claim 1, where
Y is nitrogen or oxygen;
R¹ is hydrogen;
R² is a C₃-C₁₅-carbocyclic group optionally substituted by halo, C₁-C₈-allcyl or by C₁-C₈-alkoxy optionally substituted by a 5- or 6-membered heterocyclic group containing at least one ring heteroatom selected from nitrogen, oxygen and sulphur; and
R³, R⁴ and R⁵ are independently hydrogen, halo, cyano, hydroxy, carboxy or -SO₂NH₂,
or two of R³, R⁴ and R⁵ that are attached to adjacent ring carbon atoms together form a 5- or 6-membered heterocyclic group containing at least one ring heteroatom selected from nitrogen, oxygen and sulphur.

5. A compound according to claim 1, where
Y is nitrogen or oxygen;
R¹ is hydrogen;
R² is a C₃-C₁₀-carbocyclic group, preferably phenyl or naphthyl, optionally substituted by halo, C₁-C₄-alkyl or by C₁-C₄-alkoxy optionally substituted by a 5- or 6-membered heterocyclic group containing at least one ring heteroatom selected from nitrogen, oxygen and sulphur; and R³, R⁴ and R⁵ are independently hydrogen, halo, cyano, hydroxy, carboxy or -SO₂NH₂,
or two of R³, R⁴ and R⁵ that are attached to adjacent ring carbon atoms together form a 5- or 6-membered heterocyclic group containing at least one ring heteroatom selected from nitrogen, oxygen and sulphur.

6. A compound according to claim 1 that is also a compound of formula V 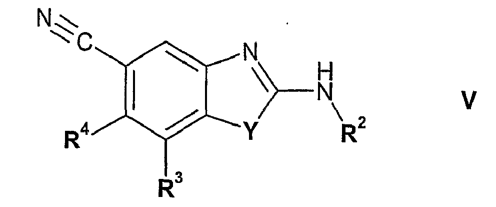 wherein Y, R², R³, and R⁴ are as shown in the following table:
| | **Y** | **R³** | **R⁴** |
|---|---|---|---|
| 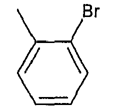 | N | -OH | -H |
| 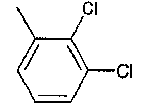 | N | -OH | -H |
| 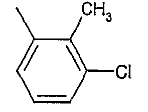 | N | -OH | -H |
| 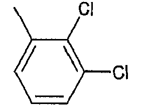 | N | -COOH | -H |
| 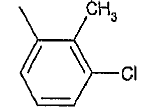 | N | -COOH | -H |
| 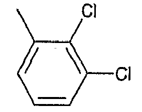 | N | -OH | -SO₂-NH₂ |
| 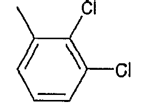 | N | 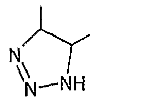 | |
| 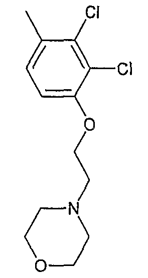 | N | -OH | -H |
| 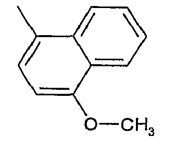 | N | -OH | -H |
| 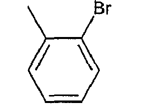 | O | -OH | -H |
| 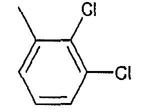 | O | -OH | -H |
| 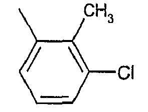 | O | -OH | -H |
| 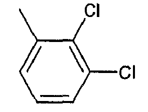 | O | -COOH | -H |
| 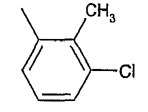 | O | -COOH | -H |

7. A compound according to claim 1 that is as shown in the following table:
| |
|---|
| 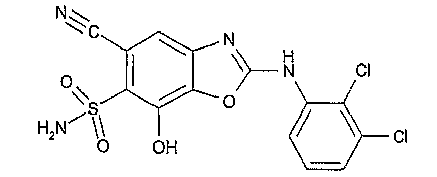 |
| 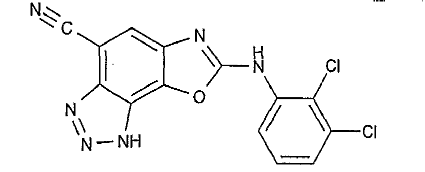 |
| 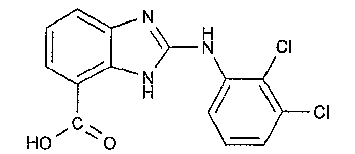 |
| 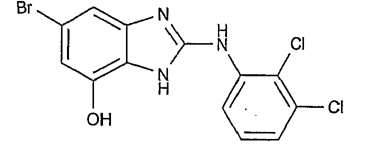 |
| 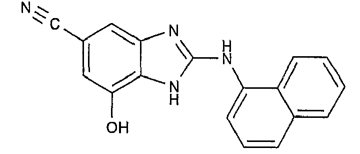 |
| 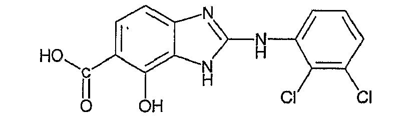 |
| 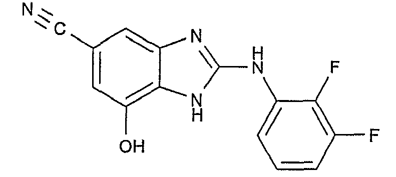 |
| 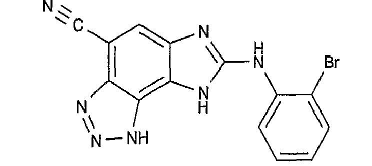 |
| 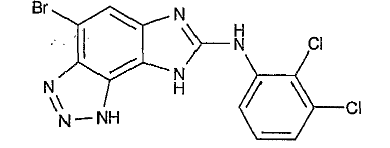 |
| 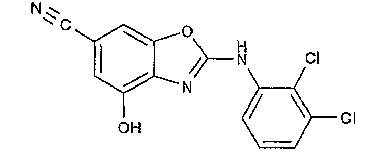 |
| 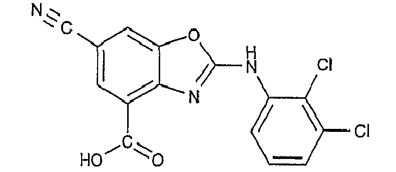 |
| 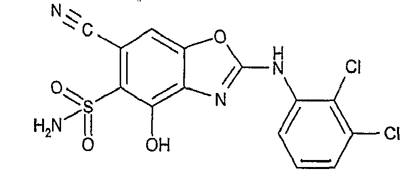 |
| 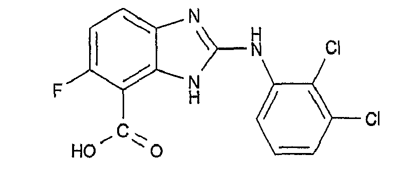 |
| 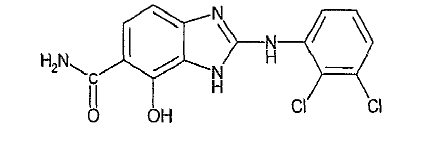 |
| 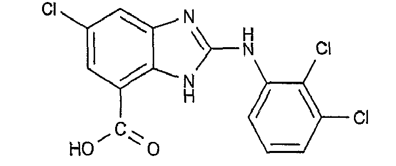 |
| 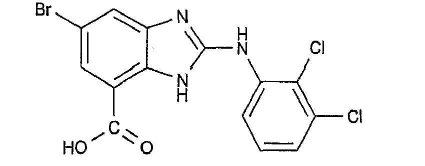 |
| 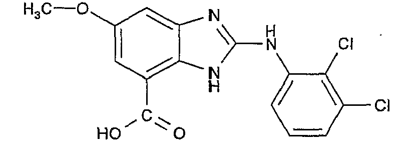 |
| 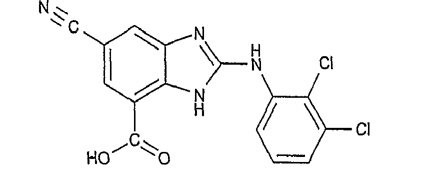 |

8. A compound according to any one of the preceding claims in combination with another drug substance which is an anti-inflammatory, a bronchodilator or an antihistamine.

9. A compound according to any one of the preceding claims for use as a pharmaceutical.

10. A pharmaceutical composition comprising as active ingredient a compound according to any one of claims 1 to 7.

11. The use of a compound according to any one of claims 1 to 7 for the manufacture of a medicament for the treatment of a condition mediated by the CXCR2 receptor.

12. The use of a compound according to any one of claims 1 to 7 for the manufacture of a medicament for the treatment of an inflammatory or allergic condition, particularly an inflammatory or obstructive airways disease.

13. A process for the preparation of a compound of formula I as claimed in claim 1 which comprises
(i)
(A) for the preparation of compounds of formula I where Y is nitrogen, cyclising a compound of formula IIa or IIb in either case optionally in protected form 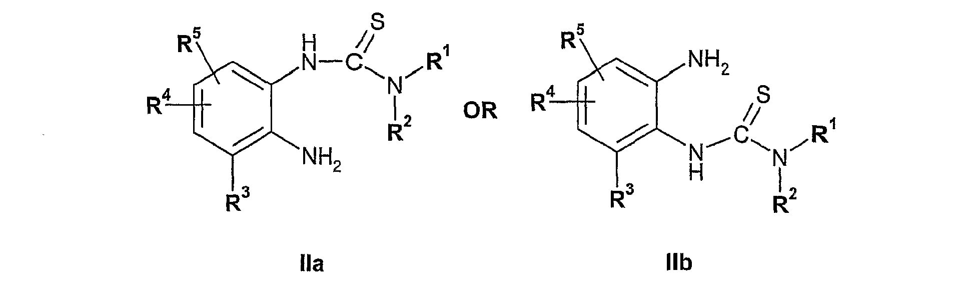 where R¹, R², R³, R⁴ and R⁵ are as defined in claim 1, and deprotecting where necessary; or
(B) for the preparation of compounds of formula I where Y is oxygen, cyclising a compound of formula IIc optionally in protected form 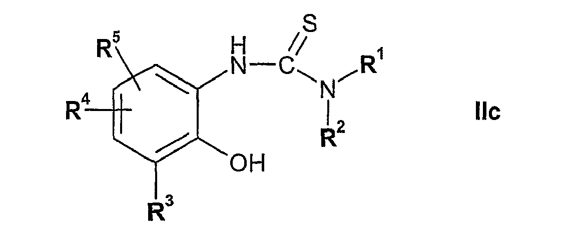 where R¹, R², R³, R⁴ and R⁵ are as defined in claim 1, and deprotecting where necessary; and
(ii) recovering the product in free or salt form.
